# EUROPEAN PATENT SPECIFICATION

(11) **EP 4 202 507 B1**
(45) Date of publication and mention of the grant of the patent: **10.06.2026**
(21) Application number: 21858360.7
(22) Date of filing: 19.08.2021
(51) Int. Cl.: G02B 5/26, G02B 5/28, C09D 1/00

(54) **OPTICAL FILTER, METHOD FOR PRODUCING SAME AND STERILIZATION DEVICE**
OPTISCHER FILTER, VERFAHREN ZUR HERSTELLUNG DAVON UND STERILISATIONSVORRICHTUNG
FILTRE OPTIQUE, PROCÉDÉ POUR SA PRODUCTION ET DISPOSITIF DE STÉRILISATION

(30) Priority: 20.08.2020 JP 2020139174; 11.12.2020 JP 2020205684; 16.06.2021 JP 2021100430
(43) Date of publication of application: 28.06.2023
(73) Proprietor: Nippon Electric Glass Co., Ltd., Otsu-shi, Shiga 520-8639 (JP)
(72) Inventor: IMAMURA, Tsutomu, Otsu-shi, Shiga 520-8639 (JP); SAHARA, Keiichi, Otsu-shi, Shiga 520-8639 (JP); NAKAMURO, Tomoyoshi, Otsu-shi, Shiga 520-8639 (JP); IMURA, Masaaki, Otsu-shi, Shiga 520-8639 (JP)
(74) Representative: Tetzner, Michael
(86) International application number: PCT/JP2021/030344
(87) International publication number: WO 2022/039216

(56) References cited:
- WO-A1-2021/043554
- WO-A1-2022/046641
- WO-A1-2022/148746
- JP-A- 2003 139 945
- JP-A- 2013 068 885
- JP-A- 2014 032 213
- JP-A- H02 192 189
- US-A1- 2018 169 279
- US-A1- 2018 202 071
- US-A1- 2019 192 708

## Description

The present invention relates to an optical filter capable of selectively transmitting light in a specific wavelength range, a method for producing the optical filter, and a sterilization device using the optical filter.

### Background Art

Optical filters capable of selectively transmitting light in a specific wavelength range have been widely used for various applications. As such optical filters, bandpass filters using dielectric films are known.

For example, JP 2013-068885 A cited below discloses a bandpass filter formed in such a manner that transmittance is largest with respect to specific ultraviolet light having a wavelength of 250 nm or less. In the bandpass filter of JP 2013-068885 A, upper and lower sides of a cavity layer formed of a dielectric film are covered with metal thin films. JP 2013-068885 A describes that the metal thin films have such a film thickness that the transmittance of light having a wavelength in the visible range is 10% or less in the bandpass filter. JP 2013-068885 A also describes that the cavity layer is formed of a dielectric film, and as the dielectric material, silicon dioxide, lanthanum fluoride, magnesium fluoride, aluminum oxide, hafnium oxide, or the like is used.

US 2019/192708 A1 describes an optical filter according to the preamble of claim 1. US 2018/169279 A1 describes an apparatus for generating at least one radiation, comprising a radiation source first arrangement configured to generate the at least one radiation having one or more wavelengths that are configured to selectively harm or damage at least one virus at least one of on a surface or in an aerosol, and at least one filter second arrangement configured to substantially prevent the at least one radiation from having any wavelength that is substantially harmful to cells of the body range. JP 2014-032213 A describes an optical functional film for infrared rays of a multilayer structure having a plurality of optical functional layers on a substrate, at least one intermediate layer between the plurality of optical functional layers comprising an adhesion reinforcing layer with a smaller thickness than any layer of the plurality of optical functional layers, wherein the adhesion reinforcing layer is a YO layer or HfO layer.

### Summary of Invention

### Technical Problem

In sterilization treatment by ultraviolet light such as sterilization treatment against bacteria and viruses adhering to the skin or the like, an excimer lamp configured to emit ultraviolet light having a wavelength from 220 nm to 225 nm, or the like is used. However, the excimer lamp has a problem: ultraviolet light having a wavelength from 240 nm to 320 nm, which is harmful light to the human body, is slightly emitted therefrom.

Even in the case of using a bandpass filter as in JP 2013-068885 A, it is difficult to sufficiently suppress the transmission of such ultraviolet light in a wavelength range from 240 nm to 320 nm. In particular, when it is attempted to suppress the transmission of ultraviolet light in a wavelength range from 240 nm to 320 nm, it is difficult, on the other hand, to sufficiently transmit ultraviolet light in a wavelength range from 220 nm to 225 nm, and thus, there arises a problem: it is difficult to suppress the transmission of the ultraviolet light harmful to the human body and to highly efficiently transmit the ultraviolet light useful for sterilization treatment at the same time at a high level.

An object of the present invention is to provide an optical filter capable of effectively transmitting ultraviolet light in a wavelength range from 220 nm to 225 nm while suppressing the transmission of ultraviolet light in a wavelength range from 240 nm to 320 nm, a method for producing the optical filter, and a sterilization device using the optical filter.

### Solution to Problem

The present invention provides an optical filter according to claim 1, a method for producing an optical filter according to claim 12, and a sterilization device according to claim 14. Preferred embodiments are subject-matter of the dependent claims. An optical filter according to the present invention, includes a transparent substrate and a dielectric multilayer film provided on the transparent substrate and containing hafnium oxide. A minimum value of spectral transmittance in a wavelength range from 220 nm to 225 nm is 50% or more with an incident angle of 0 degrees, and a maximum value of spectral transmittance in a wavelength range from 240 nm to 320 nm is 5% or less with an incident angle of 0 degrees.

In the present invention, the dielectric multilayer film contains a cubic hafnium oxide crystal.

In the present invention, in X-ray diffraction measurement, a diffraction peak by a (1 1 1) crystal plane derived from a cubic hafnium oxide crystal is preferably larger than a diffraction peak by a (-1 1 1) crystal plane derived from a monoclinic hafnium oxide crystal.

In the present invention, the dielectric multilayer film includes a high refractive index film having a relatively high refractive index and a low refractive index film having a relatively low refractive index, and the high refractive index film preferably contains the hafnium oxide. The low refractive index film more preferably contains silicon oxide.

In the present invention, the outermost layer of the dielectric multilayer film is preferably a film containing the hafnium oxide. The thickness of the outermost layer is more preferably 1 nm or more and 10 nm or less.

In the present invention, it is preferable that a ratio (T₃₀/T₀) of spectral transmittance T₃₀ at a wavelength of 222 nm with an incident angle of 30 degrees to spectral transmittance T₀ at a wavelength of 222 nm with an incident angle of 0 degrees be 0.5 or more.

In the present invention, the dielectric multilayer film includes a high refractive index film having a relatively high refractive index and a low refractive index film having a relatively low refractive index, and a ratio (t_{H}/t_{L}) of a total thickness t_{H} of the high refractive index film to a total thickness t_{L} of the low refractive index film is preferably 0.2 or more. The ratio (t_{H}/t_{L}) of the total thickness t_{H} of the high refractive index film to the total thickness t_{L} of the low refractive index film is more preferably 0.5 or more.

In the present invention, with an incident angle of 0 degrees, it is preferable that the minimum value of the spectral transmittance in a wavelength range from 220 nm to 225 nm be 50% or more, and the maximum value of the spectral transmittance in a wavelength range from 237 nm to 280 nm be 10% or less.

In the present invention, with an incident angle of 40 degrees, it is preferable that the maximum value of the spectral transmittance in a wavelength range from 237 nm to 280 nm be 20% or less.

In the present invention, a method for producing an optical filter is a method for producing an optical filter. The method includes producing a transparent substrate with a film by depositing a dielectric multilayer film containing hafnium oxide on the transparent substrate by sputtering method, and heating the transparent substrate with the film at a temperature of 500°C or higher.

In the present invention, the temperature for the heating of the transparent substrate with the film is preferably 800°C or lower.

A sterilization device according to the present invention is a device for performing inactivation treatment on microorganisms to be treated, and includes a light source configured to emit light whose wavelength is in a wavelength range from 190 nm to 230 nm, and the optical filter constituted in accordance with the present invention.

### Advantageous Effects of Invention

According to the present invention, an optical filter capable of effectively transmitting ultraviolet light in a wavelength range from 220 nm to 225 nm while suppressing the transmission of ultraviolet light in a wavelength range from 240 nm to 320 nm, a method for producing the optical filter, and a sterilization device using the optical filter may be provided.

### Brief Description of Drawings

FIG. 1 is a schematic cross-sectional view illustrating an optical filter according to a first embodiment of the present invention.
FIG. 2 is a diagram illustrating X-ray diffraction spectra of optical filters produced in Example 2 and Comparative Example 1.
FIG. 3 is a diagram illustrating transmission spectra of optical filters produced in Example 2 and Comparative Example 1.
FIG. 4 is a schematic cross-sectional view illustrating an optical filter according to a second embodiment of the present invention.
FIG. 5 is a diagram illustrating transmission spectra of an optical filter produced in Example 5 before and after the filter being immersed in hydrofluoric acid.
FIG. 6 is a diagram illustrating transmission spectra of an optical filter produced in Example 8 before and after the filter being immersed in hydrofluoric acid.
FIG. 7 is a schematic diagram illustrating a sterilization device according to an embodiment of the present invention.
FIG. 8 is a diagram illustrating transmission spectra with respective incident angles of an optical filter produced in Example 18.

### Description of Embodiments

Preferred embodiments are described below. However, the following embodiments are merely examples, and the present invention is not limited to the following embodiments. In each of the drawings, members having substantially the same function may be denoted by the same reference sign.

### First Embodiment

### Optical Filter

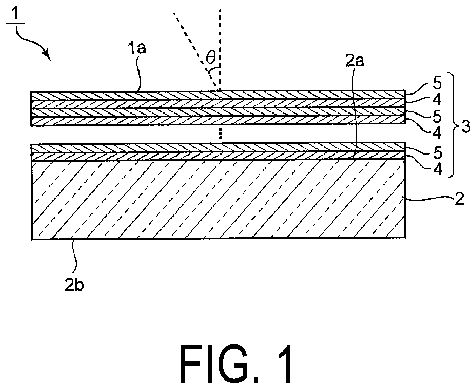

FIG. 1 is a schematic cross-sectional view illustrating an optical filter according to a first embodiment of the present invention. As illustrated in FIG. 1, an optical filter 1 includes a transparent substrate 2 and a dielectric multilayer film 3. The dielectric multilayer film 3 is provided on the transparent substrate 2.

In the present embodiment, the transparent substrate 2 has a rectangular plate shape. The transparent substrate 2 may have a shape such as a disc shape, for example, and the shape thereof is not particularly limited.

The thickness of the transparent substrate 2 is not particularly limited, and may be appropriately set in accordance with light transmittance or the like. The thickness of the transparent substrate 2 may be approximately from 0.1 mm to 30 mm, for example.

The transparent substrate 2 is preferably a substrate that is transparent in a usage wavelength range of the optical filter 1. More specifically, in the transparent substrate 2, it is preferable for the average light transmittance of the ultraviolet wavelength range to be 80% or more in a wavelength range from 220 nm to 225 nm.

The material of the transparent substrate 2 is not particularly limited, and examples thereof include glass, resin and the like. Examples of the glass include quartz glass, borosilicate glass and the like. The quartz glass may be synthetic quartz glass or molten quartz glass. The borosilicate glass preferably contains 55% to 75% of SiO₂, 1% to 10% of Al₂O₃, 10% to 30% of B₂O₃, 0% to 5% of CaO, 0% to 5% of BaO and 1.0% to 15% of (Li₂O + Na₂O + K₂O), and more preferably further contains 0% to 0.001% of TiO₂, 0% to 0.001% of Fe₂O₃ and 0.5% to 2.0% of F, as glass composition in terms of mass%.

The transparent substrate 2 has a first principal surface 2a and a second principal surface 2b opposing each other. The dielectric multilayer film 3 is provided as a filter portion on the first principal surface 2a of the transparent substrate 2.

The dielectric multilayer film 3 is a multilayer film including a high refractive index film 4 having a relatively high refractive index and a low refractive index film 5 having a relatively low refractive index. In the present embodiment, the high refractive index film 4 and the low refractive index film 5 are alternately laminated in that order on the first principal surface 2a of the transparent substrate 2 to constitute the multilayer film.

The high refractive index film 4 is formed of hafnium oxide, and is a film including hafnium oxide as a main ingredient. In the present specification, a film including a material as a main ingredient refers to a film including 50 mass% or more of the material in the film. It goes without saying that the film may contain 100 mass% of the material therein.

In the present embodiment, the low refractive index film 5 is formed of silicon oxide, and is a film including the silicon oxide as a main ingredient. The low refractive index film 5 may be a film including aluminum oxide, zirconium oxide, magnesium fluoride, silicon nitride, or the like as a main ingredient. One type of these materials may be used alone or multiple types thereof may be used together for the low refractive index film 5.

The thickness of one layer of the high refractive index film 4 is not particularly limited, but is preferably 5 nm or more and more preferably 10 nm or more, and preferably 60 nm or less and more preferably 50 nm or less.

The thickness of one layer of the low refractive index film 5 is not particularly limited, but is preferably 5 nm or more and more preferably 10 nm or more, and preferably 80 nm or less and more preferably 60 nm or less.

The entire thickness of the dielectric multilayer film 3 is not particularly limited, but is preferably 1000 nm or more and more preferably 1500 nm or more, and preferably 3000 nm or less and more preferably 2000 nm or less.

The number of layers of the film constituting the dielectric multilayer film 3 is preferably 20 or more and more preferably 30 or more, and preferably 100 or less and more preferably 80 or less.

The optical filter 1 of the present embodiment is a bandpass filter designed to selectively transmit light in a specific wavelength range by utilizing interference of light with the above-discussed dielectric multilayer film 3 included in the optical filter. Specifically, the bandpass filter is designed in such a manner that the minimum value of the spectral transmittance in a wavelength range from 220 nm to 225 nm is 50% or more, and the maximum value of the spectral transmittance in a wavelength range from 240 nm to 320 nm is 5% or less.

In this specification, the spectral transmittance may be determined by measuring the spectral transmittance of the entire optical filter 1 with a spectral transmittance meter (manufactured by Hitachi High-Tech Corporation, Product Number "UH4150"), for example. The measurement conditions may be such that the measurement is performed from the side of a principal surface 1a of the optical filter 1, the incident angle is 0 degrees, and the measurement wavelength is from 190 nm to 400 nm, for example.

As described above, the features of the optical filter 1 according to the present embodiment are such that the dielectric multilayer film 3 containing hafnium oxide is provided on the transparent substrate 2, the minimum value of the spectral transmittance in a wavelength range from 220 nm to 225 nm is 50% or more, and the maximum value of the spectral transmittance in a wavelength range from 240 nm to 320 nm is 5% or less. In this case, an incident angle θ to be described below is 0 degrees.

This allows the optical filter 1 to effectively transmit ultraviolet light in a wavelength range from 220 nm to 225 nm while suppressing the transmission of ultraviolet light in a wavelength range from 240 nm to 320 nm.

As described above, the optical filter 1 can suppress the transmission of ultraviolet light in a wavelength range from 240 nm to 320 nm, and thus can suppress the transmission of ultraviolet light harmful to the human body. Since the transmittance of ultraviolet light in a wavelength range from 220 nm to 225 nm is excellent, ultraviolet light useful for sterilization treatment such as skin sterilization treatment can be effectively transmitted.

Therefore, the optical filter 1 of the present embodiment, for example, when used together with an ultraviolet irradiation device such as an excimer lamp, can effectively transmit ultraviolet light useful for sterilization treatment while suppressing the transmission of ultraviolet light harmful to the human body.

In the present invention, the minimum value of the spectral transmittance in a wavelength range from 220 nm to 225 nm is preferably 60% or more, and more preferably 70% or more. In this case, ultraviolet light useful for sterilization treatment such as skin sterilization treatment can be more effectively transmitted. The upper limit value of the minimum value of the spectral transmittance in a wavelength range from 220 nm to 225 nm is not particularly limited, but may be, for example, 95%. In this case, an incident angle θ to be described below is 0 degrees.

In the present invention, the maximum value of the spectral transmittance in a wavelength range from 240 nm to 320 nm is preferably 3% or less, more preferably 2.5% or less, and further preferably 1% or less. In this case, the transmission of ultraviolet light harmful to the human body can be more effectively suppressed. The lower limit value of the maximum value of the spectral transmittance in a wavelength range from 240 nm to 320 nm is not particularly limited, but may be, for example, 0.2%. In this case, an incident angle θ to be described below is 0 degrees.

In the optical filter 1 of the present embodiment, it is preferable that the ratio (T₃₀/T₀) of spectral transmittance T₃₀ at a wavelength of 222 nm with an incident angle of 30 degrees to spectral transmittance T₀ at a wavelength of 222 nm with an incident angle of 0 degrees be 0.5 or more. When a lamination direction (thickness direction) of the dielectric multilayer film 3 orthogonal to a direction along the principal surface 1a of the optical filter 1 is taken as a normal direction, the incident angle refers to an angle inclined with respect to the normal direction (for example, θ in FIG. 1). Accordingly, the direction along the normal direction takes an incident angle of 0 degrees.

In this case, the spectral transmittance may be determined by measuring the spectral transmittance of the entire optical filter 1 with a spectral transmittance meter (manufactured by Hitachi High-Tech Corporation, Product Number "UH4150"), for example. The measurement conditions may be such that the measurement is performed from the principal surface 1a side of the optical filter 1, and the measurement wavelength is from 190 nm to 400 nm, for example.

In the optical filter 1 of the present embodiment, since the ratio (T₃₀/T₀) of the spectral transmittance T₃₀ at a wavelength of 222 nm with the incident angle of 30 degrees to the spectral transmittance T₀ at the wavelength of 222 nm with the incident angle of 0 degrees is the above-described lower limit value or more, ultraviolet light useful for sterilization treatment can be more efficiently transmitted even when the incident angle of the emitted light from the light source is large. This makes it possible to further increase an effective irradiation area of the emitted light from the light source.

In the present invention, the ratio (T₃₀/T₀) of the spectral transmittance T₃₀ at a wavelength of 222 nm with an incident angle of 30 degrees to the spectral transmittance T₀ at a wavelength of 222 nm with an incident angle of 0 degrees is preferably from 0.6 or more, more preferably 0.7 or more, further preferably 0.8 or more, particularly preferably 0.9 or more, and preferably 1.0 or less. When the ratio (T₃₀/T₀) falls within the above range, ultraviolet light useful for sterilization treatment can be more efficiently transmitted even when the incident angle of the emitted light from the light source is large.

In the present invention, the spectral transmittance T₀ at a wavelength of 222 nm with the incident angle of 0 degrees is preferably 60% or more, more preferably 70% or more, further preferably 75% or more, and particularly preferably 80% or more. In this case, ultraviolet light useful for sterilization treatment can be more efficiently transmitted. The upper limit value of the spectral transmittance T₀ at the wavelength of 222 nm with the incident angle of 0 degrees is more preferred as it is higher, and may be set to be 95%, for example.

The spectral transmittance T₃₀ at a wavelength of 222 nm with an incident angle of 30 degrees is preferably 40% or more, more preferably 50% or more, further preferably 60% or more, and particularly preferably 70% or more. In this case, an effective irradiation area of the emitted light from the light source can be further increased. The upper limit value of the spectral transmittance T₃₀ at the wavelength of 222 nm with the incident angle of 30 degrees is more preferred as it is higher, and may be set to be 93%, for example.

The spectral transmittance at a wavelength of 222 nm with an incident angle of 40 degrees is preferably 5% or more, more preferably 10% or more, further preferably 20% or more, and particularly preferably 30% or more. In this case, an effective irradiation area of the emitted light from the light source can be further increased. The upper limit value of the spectral transmittance at the wavelength of 222 nm with the incident angle of 40 degrees is more preferred as it is higher, and may be set to be 55%, for example.

In the present invention, with incident angle of 0 degrees, it is preferable that the minimum value of the spectral transmittance in a wavelength range from 220 nm to 225 nm be 50% or more, and the maximum value of the spectral transmittance in a wavelength range from 237 nm to 280 nm be 10% or less. In this case, it is possible to highly efficiently transmit ultraviolet light useful for sterilization treatment and to suppress the transmission of ultraviolet light harmful to the human body at the same time at a higher level.

The maximum value of the spectral transmittance in a wavelength range from 237 nm to 280 nm with an incident angle of 0 degrees is preferably 10% or less, more preferably 5% or less, further preferably 4% or less, particularly preferably 3% or less, and most preferably 2% or less. In this case, the transmission of ultraviolet light harmful to the human body can be further suppressed. The lower limit value of the maximum value of the spectral transmittance in a wavelength range from 237 nm to 280 nm with the incident angle of 0 degrees is more preferred as it is lower, and may be set to be 0.01%, for example.

The maximum value of the spectral transmittance in a wavelength range from 237 nm to 280 nm with an incident angle of 30 degrees is preferably 15% or less, more preferably 10% or less, further preferably 5% or less, particularly preferably 4% or less, and most preferably 3% or less. In this case, an effective irradiation area of the emitted light from the light source can be further increased. The lower limit value of the maximum value of the spectral transmittance in a wavelength range from 237 nm to 280 nm with the incident angle of 30 degrees is more preferred as it is lower, and may be set to be 0.01%, for example.

The maximum value of the spectral transmittance in a wavelength range from 237 nm to 280 nm with an incident angle of 40 degrees is preferably 20% or less, more preferably 15% or less, further preferably 10% or less, particularly preferably 7% or less, and most preferably 5% or less. In this case, an effective irradiation area of the emitted light from the light source can be further increased. The lower limit value of the maximum value of the spectral transmittance in a wavelength range from 237 nm to 280 nm with the incident angle of 40 degrees is more preferred as it is lower, and may be set to be 0.01%, for example.

+The spectral transmittance at a wavelength of 222 nm with each incident angle and the spectral transmittance in a wavelength range from 237 nm to 280 nm with each incident angle can be adjusted by, for example, the film configuration of the dielectric multilayer film 3.

In the present invention, a total thickness t_{H} of the high refractive index films 4 is preferably 250 nm or more, more preferably 300 nm or more, further preferably 400 nm or more and particularly preferably 500 nm or more, and preferably 1000 nm or less, more preferably 800 nm or less, further preferably 700 nm or less and particularly preferably 600 nm or less. With the total thickness t_{H} of the high refractive index films 4 being the above-described lower limit value or more, the high spectral transmittance at the wavelength of 222 nm can be maintained more effectively even when the incident angle is large. The maximum value of the spectral transmittance in a wavelength range from 237 nm to 280 nm may be further decreased. On the other hand, in a case where the total thickness t_{H} of the high refractive index films 4 is the above-described upper limit value or less, the spectral transmittance at the wavelength of 222 nm may be further increased.

The thickness of one layer of the high refractive index film 4 is not particularly limited, but is preferably 5 nm or more and more preferably 10 nm or more, and preferably 60 nm or less and more preferably 50 nm or less.

In the present invention, a total thickness t_{L} of the low refractive index films 5 is preferably 500 nm or more, more preferably 600 nm or more, further preferably 700 nm or more and particularly preferably 800 nm or more, and preferably 2000 nm or less, more preferably 1700 nm or less, further preferably 1500 nm or less and particularly preferably 1400 nm or less. In a case where the total thickness t_{L} of the low refractive index films 5 is the above-described lower limit value or more, the maximum value of the spectral transmittance in a wavelength range from 237 nm to 280 nm may be further lowered. On the other hand, in a case where the total thickness t_{L} of the low refractive index films 5 is the above-described upper limit value or less, the spectral transmittance at the wavelength of 222 nm may be further increased.

The thickness of one layer of the low refractive index film 5 is not particularly limited, but is preferably 5 nm or more and more preferably 10 nm or more, and preferably 80 nm or less and more preferably 60 nm or less.

In the present invention, a ratio (t_{H}/t_{L}) of the total thickness t_{H} of the high refractive index films 4 to the total thickness t_{L} of the low refractive index films 5 is preferably 0.2 or more, more preferably 0.3 or more, further preferably 0.4 or more, particularly preferably 0.5 or more and most preferably 0.6 or more, and preferably 1 or less, more preferably 0.9 or less, further preferably 0.8 or less and particularly preferably 0.75 or less. In a case where the ratio (t_{H}/t_{L}) is the above-described lower limit value or more, the high spectral transmittance at the wavelength of 222 nm can be maintained more effectively even when the incident angle is large. In a case where the ratio (t_{H}/t_{L}) is the above-described upper limit value or less, the maximum value of the spectral transmittance in a wavelength range from 237 nm to 280 nm may be further lowered.

The total thickness of the dielectric multilayer film 3 is not particularly limited, but is preferably 800 nm or more, more preferably 1000 nm or more, further preferably 1100 nm or more and particularly preferably 1200 nm or more, and preferably 2500 nm or less, more preferably 2200 nm or less, further preferably 2000 nm or less and particularly preferably 1900 nm or less. In a case where the total thickness of the dielectric multilayer film 3 is the above-described lower limit value or more, the maximum value of the spectral transmittance in a wavelength range from 237 nm to 280 nm may be further decreased. On the other hand, in a case where the total thickness of the dielectric multilayer film 3 is the above-described upper limit value or less, the spectral transmittance at the wavelength of 222 nm may be further increased.

The number of layers of the film constituting the dielectric multilayer film 3 is preferably 20 or more, more preferably 25 or more, further preferably 30 or more and particularly preferably 35 or more, and preferably 100 or less, more preferably 80 or less, further preferably 60 or less and particularly preferably 45 or less. In a case where the number of layers of the film constituting the dielectric multilayer film 3 is the above-described lower limit value or more, the maximum value of the spectral transmittance in a wavelength range from 237 nm to 280 nm may be further decreased. In a case where the number of layers of the film constituting the dielectric multilayer film 3 is the above-described upper limit value or less, the spectral transmittance at the wavelength of 222 nm may be further increased.

In the present invention, the dielectric multilayer film 3 contains a cubic hafnium oxide crystal. More specifically, the high refractive index film 4 constituting the dielectric multilayer film 3 preferably contains a hafnium oxide crystal, and more preferably contain a cubic hafnium oxide crystal. In this case, the transmittance of ultraviolet light in a wavelength range from 220 nm to 225 nm can be further increased while the transmission of ultraviolet light in a wavelength range from 240 nm to 320 nm is further suppressed.

In the present specification, whether the cubic hafnium oxide crystal is contained may be confirmed by whether the diffraction peak by the (1 1 1) crystal plane derived from the cubic hafnium oxide crystal is observed in the X-ray diffraction measurement.

In the present specification, the X-ray diffraction measurement may be performed by a wide angle X-ray diffraction technique. As an X-ray diffractometer, for example, "SmartLab", which is a product of Rigaku Corporation, may be used. As a radiation source, CuKα radiation may be used. In the X-ray diffraction measurement as well, the entire optical filter 1 is subjected to measurement from the first principal surface 2a side.

In the present invention, in the X-ray diffraction measurement, a diffraction peak by a (1 1 1) crystal plane derived from a cubic hafnium oxide crystal is preferably larger than a diffraction peak by a (-1 1 1) crystal plane derived from a monoclinic hafnium oxide crystal. In this case, the transmittance of ultraviolet light in a wavelength range from 220 nm to 225 nm can be further increased while the transmission of ultraviolet light in a wavelength range from 240 nm to 320 nm is further suppressed.

In the present invention, a ratio Ic/Im of a peak integrated intensity Ic of the diffraction peak by the (1 1 1) crystal plane derived from a cubic hafnium oxide crystal to a peak integrated intensity Im of the diffraction peak by the (-1 1 1) crystal plane derived from a monoclinic hafnium oxide crystal is preferably 0.1 or more, more preferably 0.3 or more, further preferably 1 or more, still preferably 2 or more, particularly preferably 2.5 or more, and most preferably 3 or more. In a case where the ratio Ic/Im is the above-described lower limit value or more, the transmittance of ultraviolet light in a wavelength range from 220 nm to 225 nm can be further increased while the transmission of ultraviolet light in a wavelength range from 240 nm to 320 nm is further suppressed. The upper limit value of the ratio Ic/Im is not particularly limited, but may be set to be, for example, 10000.

In the present invention, an anti-reflection film may be provided on the second principal surface 2b of the transparent substrate 2. In this case, the transmittance of ultraviolet light in a wavelength range from 220 nm to 225 nm can be further enhanced.

The anti-reflection film is not particularly limited, and for example, a multilayer film including a high refractive index film having a relatively high refractive index and a low refractive index film having a relatively low refractive index may be used. The multilayer film may be constituted by the high refractive index film and the low refractive index film being alternately provided in that order. As the high refractive index film, for example, a film including hafnium oxide as a main ingredient may be used. Examples of the low refractive index film include a film containing silicon oxide, aluminum oxide, zirconium oxide, tin oxide, silicon nitride or the like as a main ingredient. The number of layers of the film constituting the multilayer film may be, for example, 4 or more and 100 or less.

In the present invention, as long as the advantageous effects of the present invention are not hindered, a film other than an anti-reflection film may be laminated on the second principal surface 2b of the transparent substrate 2. Furthermore, as long as the advantageous effects of the present invention are not hindered, a film other than the dielectric multilayer film 3 may also be provided on the first principal surface 2a of the transparent substrate 2. In this case, the film may be provided between the transparent substrate 2 and the dielectric multilayer film 3, or may be provided on the dielectric multilayer film 3.

An example of a method for producing the optical filter 1 will be described in detail below.

### Production Method for Optical Filter

### The Step of Forming Transparent Substrate with Film;

First, the transparent substrate 2 is prepared. Subsequently, the dielectric multilayer film 3 is formed on the first principal surface 2a of the transparent substrate 2. The dielectric multilayer film 3 may be formed by alternately laminating the high refractive index film 4 and the low refractive index film 5 in that order on the first principal surface 2a of the transparent substrate 2. The high refractive index film 4 and the low refractive index film 5 may each be formed by sputtering method.

The temperature of the substrate when the high refractive index film 4 is deposited is preferably 300°C or lower, and more preferably 270°C or lower. In this case, in the resultant optical filter 1, ultraviolet light having a wavelength from 220 nm to 225 nm may be transmitted more effectively while the transmission of light having a wavelength from 240 nm to 320 nm is further suppressed. The lower limit value of the temperature of the substrate when the high refractive index film 4 is deposited may be, for example, 20°C.

The deposition of the high refractive index film 4 may be carried out, for example, by using a target of a material for constituting the high refractive index film 4, setting the flow rate of an inert gas such as an argon gas as a carrier gas to 50 sccm to 500 sccm, and applying power from 0.5 kW to 40 kW.

The deposition of the low refractive index film 5 may be carried out, for example, by using a target of a material for constituting the low refractive index film 5, setting the flow rate of an inert gas such as an argon gas as a carrier gas to 50 sccm to 500 sccm, and applying power from 0.5 kW to 40 kW.

### The Step of Heating;

Next, the resultant transparent substrate with the film is heated at a temperature of 500°C or higher. As a result, the optical filter 1 may be produced. In particular, when the transparent substrate with the film is heated at a temperature of 450°C or higher, the content of the cubic hafnium oxide crystal can be made relatively larger. Due to this, in the produced optical filter 1, the transmittance of ultraviolet light in a wavelength range from 220 nm to 225 nm can be further increased while the transmission of ultraviolet light in a wavelength range from 240 nm to 320 nm is further suppressed.

The temperature for heating the transparent substrate with the film is 500°C or higher and preferably 550°C or higher, and preferably 800°C or lower and more preferably 750°C or lower. In a case where the heating temperature falls within the above-described range, the transmittance of ultraviolet light in a wavelength range from 220 nm to 225 nm can be further increased while the transmission of ultraviolet light in a wavelength range from 240 nm to 320 nm is further suppressed.

The period of time of heating the transparent substrate with the film is not particularly limited, but may be, for example, 10 minutes or more and 120 minutes or less.

In the present invention, in the X-ray diffraction measurement of the transparent substrate with the film before being heated, it is preferable that the intensity of the diffraction peak by the (-1 1 1) crystal plane derived from the monoclinic hafnium oxide crystal be small. The intensity of the diffraction peak by the (-1 1 1) crystal plane derived from the monoclinic hafnium oxide crystal is preferably at a microcrystalline level, and the height of the peak intensity is more preferably three times or less the height of the peak intensity of an amorphous halo. In this case, the ratio Ic/Im of the peak integrated intensity Ic of the diffraction peak by the (1 1 1) crystal plane derived from the cubic hafnium oxide crystal to the peak integrated intensity Im of the diffraction peak by the (-1 1 1) crystal plane derived from the monoclinic hafnium oxide crystal can be further increased by heating. Due to this, in the produced optical filter 1, the transmittance of ultraviolet light in a wavelength range from 220 nm to 225 nm can be further increased while the transmission of ultraviolet light in a wavelength range from 240 nm to 320 nm is further suppressed.

In the present invention, the transmittance of ultraviolet light in a wavelength range from 240 nm to 320 nm and the transmittance of ultraviolet light in a wavelength range from 220 nm to 225 nm may be adjusted by, for example, the total number of films constituting the dielectric multilayer film 3, the film thickness, the material, and the heating temperature of the transparent substrate with the film. In particular, in the produced optical filter 1, by the heating temperature of the transparent substrate with the film, the transmittance of ultraviolet light in a wavelength range from 220 nm to 225 nm can be further effectively increased while the transmission of ultraviolet light in a wavelength range from 240 nm to 320 nm is further suppressed.

The spectral transmittance at the wavelength of 222 nm with each incident angle, and the spectral transmittance in a wavelength range from 237 nm to 280 nm with each incident angle may also be adjusted by, for example, the heating temperature of the transparent substrate with the film in addition to the number of films constituting the dielectric multilayer film 3, the film thickness, and the material. In particular, in the produced optical filter 1, by the heating temperature of the transparent substrate with the film, the maximum value of the spectral transmittance in a wavelength range from 237 nm to 280 nm may be further lowered while the spectral transmittance at the wavelength of 222 nm is further increased.

### Second Embodiment

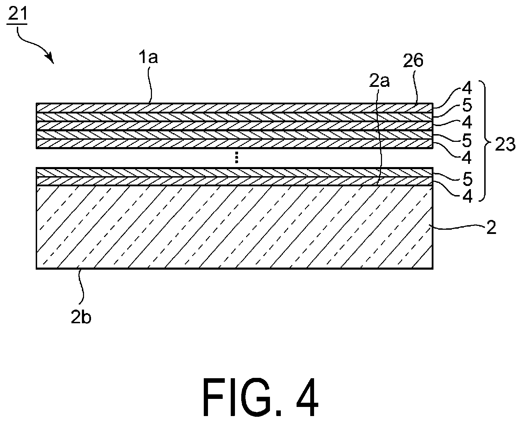

FIG. 4 is a schematic cross-sectional view illustrating an optical filter according to a second embodiment of the present invention. As illustrated in FIG. 4, in an optical filter 21, an outermost layer 26 of a dielectric multilayer film 23 is a high refractive index film 4 constituted by hafnium oxide. Other points are similar to those in the first embodiment.

In the optical filter 21 as well, the minimum value of the spectral transmittance in a wavelength range from 220 nm to 225 nm is 50% or more, and the maximum value of the spectral transmittance in a wavelength range from 240 nm to 320 nm is 5% or less. This makes it possible to effectively transmit ultraviolet light in a wavelength range from 220 nm to 225 nm while suppressing the transmission of ultraviolet light in a wavelength range from 240 nm to 320 nm.

When an excimer lamp or the like configured to emit ultraviolet light having a wavelength from 220 nm to 225 nm is used, device members may be deteriorated by the irradiation with light, and an acid gas may be generated in some cases. The gas may erode the film of the optical filter to change the optical characteristics, and thus the required characteristics cannot be realized in some cases.

In contrast, when the outermost layer 26 is constituted by hafnium oxide as in the optical filter 21, it is allowed to further suppress the erosion by the acid gas, and further suppress the change in the optical characteristics.

The thickness of the outermost layer 26 is preferably 1 nm or more and more preferably 2 nm or more, and preferably 10 nm or less and more preferably 7 nm or less. When the thickness of the outermost layer 26 is the above-described lower limit value or more, it is allowed to further suppress the erosion by the acid gas, and further suppress the change in the optical characteristics. On the other hand, when the thickness of the outermost layer 26 is the above-described upper limit value or less, ultraviolet light in a wavelength range from 220 nm to 225 nm may be further effectively transmitted while the transmission of ultraviolet light in a wavelength range from 240 nm to 320 nm is further suppressed.

### Sterilization Device

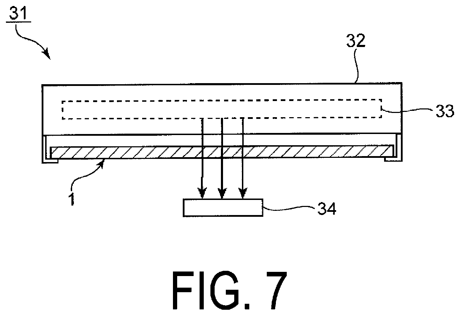

FIG. 7 is a schematic diagram illustrating a sterilization device according to an embodiment of the present invention. As illustrated in FIG. 7, a sterilization device 31 includes a housing 32, a light source 33, and the optical filter 1. The light source 33 configured to emit light whose wavelength falls within a wavelength range from 190 nm to 230 nm is disposed inside the housing 32. The light source 33 and the optical filter 1 are provided facing each other. At this time, the dielectric multilayer film 3 is preferably provided on the light source 33 side. In the sterilization device 31, a sterilization target object 34 is irradiated with the emitted light from the light source 33 via the optical filter 1.

An excimer lamp, for example, may be used as the light source 33. An example of the excimer lamp that is preferably used includes an excimer lamp configured to emit ultraviolet light having a wavelength from 220 nm to 225 nm. An example of such excimer lamp that may be used includes a KrCl excimer lamp. The excimer lamp may be a KrBr excimer lamp.

In the sterilization device 31 of the present embodiment, since the optical filter 1 described above is used, ultraviolet light useful for sterilization treatment can be efficiently transmitted. As a result, ultraviolet sterilization can be efficiently performed on the sterilization target object 34. In the ultraviolet sterilization, the ultraviolet light is made to act on DNA inside the cells of a sterilization target organism such as bacteria, thereby making it possible to selectively inactivate the target. In the ultraviolet sterilization, the ultraviolet light is also made to act on viruses to make it possible to selectively inactivate the viruses. In particular, the sterilization device 31 is more preferably used for inactivating a treatment target microorganism.

Hereinafter, the present invention will be described in more detail based on specific examples. The present invention is not limited to the following examples in any way, and can be appropriately changed and implemented within a range that does not change the gist thereof.

### Production Example 1

First, a synthetic quartz glass substrate (manufactured by USTRON Corporation) was prepared as a transparent substrate. Subsequently, a dielectric multilayer film was deposited by sputtering on a principal surface on one side of the prepared transparent substrate. Specifically, first, a hafnium target was sputtered using an argon gas and an oxygen gas as carrier gases, and thus a hafnium oxide film (HfO₂ film) was deposited on the principal surface on the one side of the transparent substrate. At this time, each of the flow rates of the argon gas and the oxygen gas was 100 sccm, and the target application power (power for deposition) was 4 kW. Subsequently, a silicon target was sputtered using the argon gas and the oxygen gas as the carrier gases, and thus a silicon oxide film (SiO₂ film) was deposited on the HfO₂ film. At this time, the flow rate of the argon gas and the oxygen gas was 100 sccm, and the target application power (power for deposition) was 4 kW. By repeating this operation, on the principal surface on the one side of the transparent substrate, the dielectric multilayer film including a film having a total of 38 layers was formed in which the HfO₂ film and the SiO₂ film were alternately laminated one layer by one layer, and thus the transparent substrate with the film was produced. The substrate temperature was maintained at room temperature (20°C) during the deposition.

### Production Example 2

A transparent substrate with a film was produced in the same manner as that in Production Example 1 except that the substrate temperature was maintained at 270°C during the deposition.

### Production Example 3

A transparent substrate with a film was produced in the same manner as that in Production Example 1 except that a molten quartz glass substrate (manufactured by USTRON Corporation) was used as a transparent substrate and the deposition was performed to cause each layer to have the film thickness as depicted in Table 1 below.

### Production Example 4

A transparent substrate with a film was produced in the same manner as that in Production Example 1 except that a borosilicate glass substrate (manufactured by Nippon Electric Glass Co., Ltd., Product Number "BU-41") was used as a transparent substrate and the deposition was performed to cause each layer to have the film thickness as depicted in Table 1 below.

The thickness of each layer in the transparent substrates with films fabricated in Production Examples 1 to 4 is as depicted in Table 1 below.

**[Table 1]**

| | | Production Example 1 | Production Example 2 | Production Example 3 | Production Example 4 | Production Example 6 |
|---|---|---|---|---|---|---|
| Glass Side | | | | | | |
| 1st Layer | HfO₂ | 37.9 nm | 37.9 nm | 36.8 nm | 36.8 nm | 35.5 nm |
| 2nd Layer | SiO₂ | 50.2 nm | 50.2 nm | 40.6 nm | 40.6 nm | 39.1 nm |
| 3rd Layer | HfO₂ | 34.7 nm | 34.7 nm | 33.5 nm | 33.5 nm | 32.2 nm |
| 4th Layer | SiO₂ | 58.4 nm | 58.4 nm | 47.9 nm | 47.9 nm | 46.2 nm |
| 5th Layer | HfO₂ | 33.9 nm | 33.9 nm | 35.0 nm | 35.0 nm | 33.7 nm |
| 6th Layer | SiO₂ | 49.4 nm | 49.4 nm | 70.9 nm | 70.9 nm | 68.4 nm |
| 7th Layer | HfO₂ | 32.0 nm | 32.0 nm | 35.4 nm | 35.4 nm | 34.1 nm |
| 8th Layer | SiO₂ | 52.3 nm | 52.3 nm | 48.9 nm | 48.9 nm | 47.1 nm |
| 9th Layer | HfO₂ | 38.0 nm | 38.0 nm | 30.9 nm | 30.9 nm | 29.8 nm |
| 10th Layer | SiO₂ | 53.8 nm | 53.8 nm | 42.5 nm | 42.5 nm | 41.0 nm |
| 11th Layer | HfO₂ | 31.5 nm | 31.5 nm | 31.3 nm | 31.3 nm | 30.2 nm |
| 12th Layer | SiO₂ | 48.0 nm | 48.0 nm | 53.5 nm | 53.5 nm | 51.6 nm |
| 13th Layer | HfO₂ | 30.0 nm | 30.0 nm | 43.2 nm | 43.2 nm | 41.6 nm |
| 14th Layer | SiO₂ | 50.5 nm | 50.5 nm | 61.5 nm | 61.5 nm | 59.3 nm |
| 15th Layer | HfO₂ | 42.0 nm | 42.0 nm | 29.3 nm | 29.3 nm | 28.2 nm |
| 16th Layer | SiO₂ | 51.3 nm | 51.3 nm | 44.0 nm | 44.0 nm | 42.4 nm |
| 17th Layer | HfO₂ | 28.9 nm | 28.9 nm | 30.5 nm | 30.5 nm | 29.4 nm |
| 18th Layer | SiO₂ | 46.1 nm | 46.1 nm | 42.7 nm | 42.7 nm | 41.2 nm |
| 19th Layer | HfO₂ | 27.3 nm | 27.3 nm | 29.2 nm | 29.2 nm | 28.2 nm |
| 20th Layer | SiO₂ | 44.6 nm | 44.6 nm | 41.8 nm | 41.8 nm | 40.3 nm |
| 21st Layer | HfO₂ | 26.4 nm | 26.4 nm | 29.8 nm | 29.8 nm | 28.7 nm |
| 22nd Layer | SiO₂ | 42.1 nm | 42.1 nm | 41.0 nm | 41.0 nm | 39.6 nm |
| 23rd Layer | HfO₂ | 25.8 nm | 25.8 nm | 29.7 nm | 29.7 nm | 28.6 nm |
| 24th Layer | SiO₂ | 42.1 nm | 42.1 nm | 41.0 nm | 41.0 nm | 39.6 nm |
| 25th Layer | HfO₂ | 26.4 nm | 26.4 nm | 29.2 nm | 29.2 nm | 28.1 nm |
| 26th Layer | SiO₂ | 43.6 nm | 43.6 nm | 41.6 nm | 41.6 nm | 40.1 nm |
| 27th Layer | HfO₂ | 26.8 nm | 26.8 nm | 28.9 nm | 28.9 nm | 27.9 nm |
| 28th Layer | SiO₂ | 43.8 nm | 43.8 nm | 42.9 nm | 42.9 nm | 41.4 nm |
| 29th Layer | HfO₂ | 26.7 nm | 26.7 nm | 28.6 nm | 28.6 nm | 27.6 nm |
| 30th Layer | SiO₂ | 42.5 nm | 42.5 nm | 42.4 nm | 42.4 nm | 40.9 nm |
| 31st Layer | HfO₂ | 26.4 nm | 26.4 nm | 29.6 nm | 29.6 nm | 28.5 nm |
| 32nd Layer | SiO₂ | 41.8 nm | 41.8 nm | 42.0 nm | 42.0 nm | 40.5 nm |
| 33rd Layer | HfO₂ | 26.8 nm | 26.8 nm | 29.0 nm | 29.0 nm | 27.9 nm |
| 34th Layer | SiO₂ | 42.2 nm | 42.2 nm | 41.1 nm | 41.1 nm | 39.7 nm |
| 35th Layer | HfO₂ | 28.5 nm | 28.5 nm | 32.7 nm | 32.7 nm | 31.5 nm |
| 36th Layer | SiO₂ | 42.9 nm | 42.9 nm | 35.8 nm | 35.8 nm | 34.5 nm |
| 37th Layer | HfO₂ | 32.8 nm | 32.8 nm | 32.4 nm | 32.4 nm | 31.2 nm |
| 38th Layer | SiO₂ | 76.9 nm | 76.9 nm | 87.1 nm | 87.1 nm | 84.0 nm |
| Air Side | | | | | | |

### Production Example 5

First, a synthetic quartz glass substrate (manufactured by USTRON Corporation) was prepared as a transparent substrate. Subsequently, a dielectric multilayer film was deposited by sputtering on a principal surface on one side of the prepared transparent substrate. Specifically, first, a hafnium target was sputtered using an argon gas and an oxygen gas as carrier gases, and thus a hafnium oxide film (HfO₂ film) was deposited on the principal surface on the one side of the transparent substrate. At this time, each of the flow rates of the argon gas and the oxygen gas was 100 sccm, and the target application power (power for deposition) was 4 kW. Subsequently, a silicon target was sputtered using the argon gas and the oxygen gas as the carrier gases, and thus a silicon oxide film (SiO₂ film) was deposited on the HfO₂ film. At this time, the flow rate of the argon gas and the oxygen gas was 100 sccm, and the target application power (power for deposition) was 4 kW. By repeating this operation, on the principal surface on the one side of the transparent substrate, the dielectric multilayer film including a film having a total of 39 layers was formed in which the HfO₂ film and the SiO₂ film were alternately laminated one layer by one layer so that the outermost layer was made of the HfO₂ film, and thus the transparent substrate with the film was produced. The substrate temperature was maintained at room temperature (20°C) during the deposition.

The thickness of each layer in the transparent substrate with the film fabricated in Production Example 5 is as depicted in Table 2 below.

**[Table 2]**

| | | Production Example 5 |
|---|---|---|
| Glass Side | | |
| 1st Layer | HfO₂ | 37 nm |
| 2nd Layer | SiO₂ | 43 nm |
| 3rd Layer | HfO₂ | 33 nm |
| 4th Layer | SiO₂ | 47 nm |
| 5th Layer | HfO₂ | 37 nm |
| 6th Layer | SiO₂ | 69 nm |
| 7th Layer | HfO₂ | 36 nm |
| 8th Layer | SiO₂ | 46 nm |
| 9th Layer | HfO₂ | 31 nm |
| 10th Layer | SiO₂ | 45 nm |
| 11th Layer | HfO₂ | 31 nm |
| 12th Layer | SiO₂ | 50 nm |
| 13th Layer | HfO₂ | 44 nm |
| 14th Layer | SiO₂ | 62 nm |
| 15th Layer | HfO₂ | 30 nm |
| 16th Layer | SiO₂ | 44 nm |
| 17th Layer | HfO₂ | 30 nm |
| 18th Layer | SiO₂ | 43 nm |
| 19th Layer | HfO₂ | 30 nm |
| 20th Layer | SiO₂ | 42 nm |
| 21st Layer | HfO₂ | 30 nm |
| 22nd Layer | SiO₂ | 41 nm |
| 23rd Layer | HfO₂ | 30 nm |
| 24th Layer | SiO₂ | 41 nm |
| 25th Layer | HfO₂ | 29 nm |
| 26th Layer | SiO₂ | 42 nm |
| 27th Layer | HfO₂ | 29 nm |
| 28th Layer | SiO₂ | 43 nm |
| 29th Layer | HfO₂ | 28 nm |
| 30th Layer | SiO₂ | 42 nm |
| 31st Layer | HfO₂ | 29 nm |
| 32nd Layer | SiO₂ | 43 nm |
| 33rd Layer | HfO₂ | 30 nm |
| 34th Layer | SiO₂ | 41 nm |
| 35th Layer | HfO₂ | 31 nm |
| 36th Layer | SiO₂ | 37 nm |
| 37th Layer | HfO₂ | 31 nm |
| 38th Layer | SiO₂ | 76 nm |
| 39th Layer | HfO₂ | 5 nm |
| Air Side | | |

### Production Example 6

A transparent substrate with a film was produced in the same manner as that in Production Example 1 except that a molten quartz glass substrate (manufactured by USTRON Corporation) was used as a transparent substrate and the deposition was performed to cause each layer to have the film thickness as depicted in Table 1 above.

### Examples 1 to 17 and Comparative Examples 1 to 4

In Example 1, an optical filter was produced by heating the transparent substrate with the film produced in Production Example 1 at a temperature of 500°C for 60 minutes under atmospheric environment. Likewise, in each of Examples 2 to 17, an optical filter was produced by heating the transparent substrate with the film produced in each of Production Examples at the temperature and for the period of time depicted in Table 3 below under atmospheric environment. As depicted in Table 3 below, in Comparative Examples 1 to 4, the transparent substrate with the film produced in each Production Example was used as it was as an optical filter without being heated.

### Evaluation

### X-Ray Diffraction Measurement

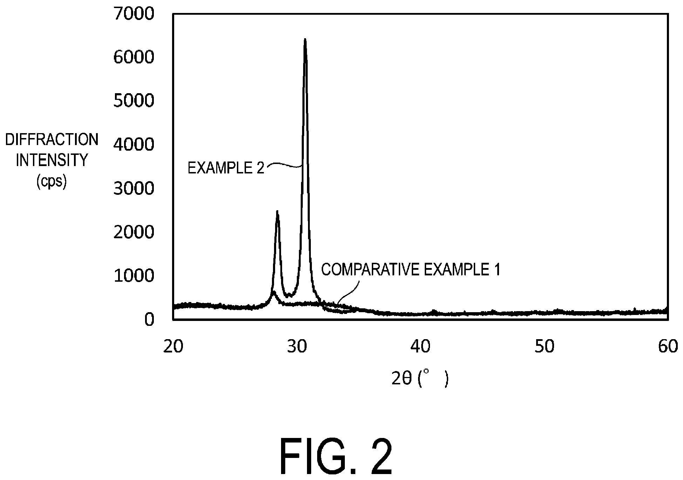

The optical filters of Examples 1 to 7 and 9 to 17 and Comparative Examples 1 to 4 were subjected to X-ray diffraction measurement using a wide angle X-ray diffraction technique. As an X-ray diffractometer, "SmartLab", which is a product of Rigaku Corporation, was used. The measurement was performed using CuKα radiation (wavelength of 1.5418 Å) as a radiation source under the conditions of a scanning axis being 2θ/ω, a measurement range being 10 degrees to 65 degrees, a scanning rate being 2 degrees/min, a tube current being 200 mA, and a tube voltage being 45 kV. An example of X-ray diffraction spectra of the produced optical filters is depicted in FIG. 2.

FIG. 2 is a diagram illustrating X-ray diffraction spectra of the optical filters produced in Example 2 and Comparative Example 1. As illustrated in FIG. 2, in the X-ray diffraction spectrum of Example 2, a diffraction peak by the (1 1 1) crystal plane derived from a cubic hafnium oxide crystal was observed in the vicinity of 2θ being 30.7 degrees, and a diffraction peak by the (-1 1 1) crystal plane derived from a monoclinic hafnium oxide crystal was observed in the vicinity of 2θ being 28.4 degrees. On the other hand, in Comparative Example 1, no diffraction peak by the (1 1 1) crystal plane derived from the cubic hafnium oxide crystal was observed.

Likewise, the optical filters of Examples 1, 3 to 7 and 9 to 17, and Comparative Examples 2 to 4 were also subjected to X-ray diffraction measurement to determine a peak integrated intensity Ic of a diffraction peak by the (1 1 1) crystal plane derived from a cubic hafnium oxide crystal, a peak integrated intensity Im of a diffraction peak by the (-1 1 1) crystal plane derived from a monoclinic hafnium oxide crystal, and a ratio Ic/Im. The results are presented in Table 3 given below.

### Spectral Transmittance

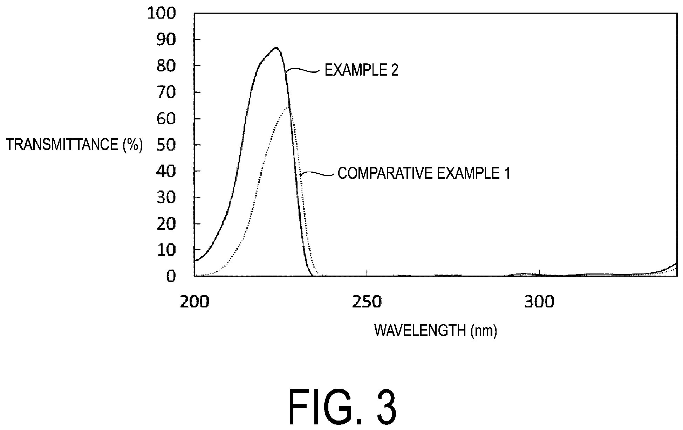

The spectral transmittance of each of the optical filters of Examples 1 to 17 and Comparative Examples 1 to 4 was measured using a spectral transmittance meter (manufactured by Hitachi High-Tech Corporation, Product Number "UH4150"). Specifically, the incident angle (angle of incidence) was set to 0 degrees, and the measurement wavelength was from 190 nm to 400 nm. An example of transmission spectra of the produced optical filters is depicted in FIG. 3.

FIG. 3 is a diagram illustrating transmission spectra of the optical filters produced in Example 2 and Comparative Example 1. As illustrated in FIG. 3, in Example 2, it can be confirmed that the minimum value of the spectral transmittance in a wavelength range from 220 nm to 225 nm was increased. On the other hand, in Comparative Example 1, the minimum value of the spectral transmittance in a wavelength range from 220 nm to 225 nm was unable to be sufficiently increased. In both Example 2 and Comparative Example 1, it is understood that the maximum value of the spectral transmittance in a wavelength range from 240 nm to 320 nm is lowered.

Likewise, as for the optical filters of Examples 1 and 3 to 17 as well as Comparative Examples 2 to 4, the spectral transmittance in a wavelength range from 220 nm to 225 nm and the spectral transmittance in a wavelength range from 240 nm to 320 nm were measured. As described above, the incident angle was set to be 0 degrees.

The results are presented in Table 3 given below.

**[Table 3]**

| | Transparent Substrate with Film | Heating Treatment | Diffraction Peak Intensity | | Peak Ratio | Wavelength Range from 220 nm to 225 nm | Wavelength Range from 240 nm to 320 nm | Transparent Substrate | Substrate Temperature |
|---|---|---|---|---|---|---|---|---|---|
| | | | Ic | Im | Ic/Im | Minimum Transmittance (%) | Maximum Transmittance (%) | | |
| Example 1 | Production Example 1 | 500°C·60 min | 4098 | 1521 | 2.69 | 84.0 | 1.0 | Synthetic quartz glass substrate (manufactured by USTRON Corporation) | Room temperature (20°C) |
| Example 2 | | 600°C·60 min | 4472 | 1783 | 2.51 | 82.3 | 0.9 | | |
| Example 3 | | 700°C·60 min | 3152 | 2030 | 1.55 | 80.1 | 1.0 | | |
| Comparative Example 1 | | Unburned | 0 | 362 | 0 | 42.2 | 0.9 | | |
| Example 4 | Production Example 2 | 600°C·60 min | 570 | 3946 | 0.14 | 62.3 | 1.2 | | 270°C |
| Comparative Example 2 | | Unburned | 0 | 2769 | 0 | 48.9 | 0.8 | | |
| Example 5 | Production Example 3 | 600°C·60 min | 3943 | 1497 | 2.63 | 77.3 | 2.4 | Molten quartz glass substrate (manufactured by USTRON Corporation) | Room temperature (20°C) |
| Comparative Example 3 | | Unburned | 0 | 235 | 0 | 40.4 | 2.2 | | |
| Example 6 | Production Example 4 | 600°C·60 min | 1070 | 6605 | 0.16 | 67.5 | 2.3 | Borosilicate glass substrate (manufactured by Nippon Electric Glass Co., Ltd., Product Number "BU-41") | |
| Example 7 | | 800°C·60 min | 401 | 5753 | 0.07 | 69.7 | 2.3 | | |
| Comparative Example 4 | | Unburned | 0 | 2612 | 0 | 45.5 | 2.1 | | |
| Example 8 | Production Example 5 | 600°C·60 min | - | - | - | 80.0 | 2.3 | Synthetic quartz glass substrate (manufactured by USTRON Corporation) | |
| Example 9 | Production Example 6 | 525°C·60 min | 7593 | 1563 | 4.86 | 73.3 | 2.4 | Molten quartz glass substrate (manufactured by USTRON Corporation) | |
| Example 10 | | 550°C·60 min | 7797 | 1730 | 4.51 | 77.8 | 2.5 | | |
| Example 11 | | 575°C·60 min | 6354 | 1561 | 4.07 | 79.2 | 2.4 | | |
| Example 12 | | 600°C·60 min | 7566 | 1979 | 3.82 | 79.5 | 2.5 | | |
| Example 13 | | 650°C·60 min | 6511 | 2064 | 3.15 | 78.9 | 2.5 | | |
| Example 14 | | 700°C·60 min | 6230 | 2395 | 2.60 | 77.6 | 2.4 | | |
| Example 15 | | 750°C·60 min | 5217 | 2981 | 1.75 | 76.3 | 2.5 | | |
| Example 16 | | 800°C·60 min | 3352 | 3946 | 0.85 | 72.5 | 2.8 | | |
| Example 17 | | 600°C·600 min | 5128 | 3309 | 1.55 | 77.0 | 2.6 | | |

As is apparent from Table 3, in the optical filters of Examples 1 to 17, ultraviolet light having a wavelength from 220 nm to 225 nm was allowed to pass through effectively while the transmission of ultraviolet light in a wavelength range from 240 nm to 320 nm is suppressed. On the other hand, in the optical filters of Comparative Examples 1 to 4, ultraviolet light having a wavelength from 220 nm to 225 nm was unable to be sufficiently transmitted.

### Erosion Test

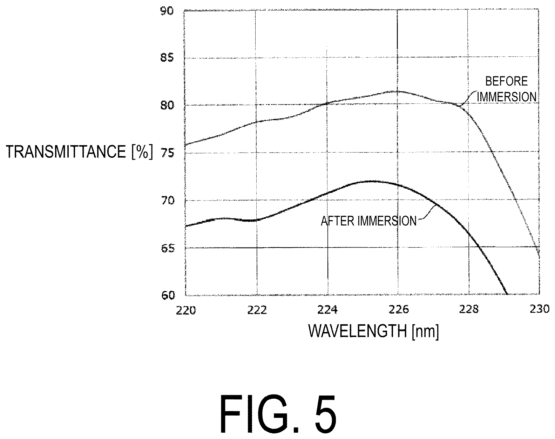

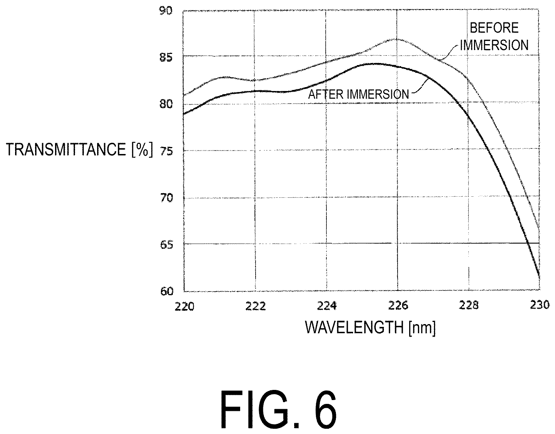

The optical filters produced in Example 5 and Example 8 were immersed in 0.5-wt.% hydrofluoric acid (HF) to carry out erosion test. Examples of transmission spectra before and after the erosion are depicted in FIGS. 5 and 6.

FIG. 5 is a diagram illustrating transmission spectra of the optical filter produced in Example 5 before and after the filter being immersed in the hydrofluoric acid. FIG. 6 is a diagram illustrating transmission spectra of the optical filter produced in Example 8 before and after the filter being immersed in the hydrofluoric acid. In FIGS. 5 and 6, the immersion time in the hydrofluoric acid was 240 seconds.

From FIGS. 5 and 6, it is understood that a change in transmittance in a wavelength range from 220 nm to 225 nm is suppressed in Example 8 with the outermost layer formed of a HfO₂ film as compared to Example 5 with the outermost layer formed of a SiO₂ film.

In Table 4 below, for the optical filters produced in Example 5 and Example 8, a relationship between the erosion amount and the change in transmittance in a wavelength range from 220 nm to 225 nm in each immersion time is depicted.

**[Table 4]**

| | Example 5 | | | Example 8 | | |
|---|---|---|---|---|---|---|
| Outermost Layer | SiO₂ | | | HfO₂ | | |
| 0.5 wt.% HF immersion time (sec) | Film thickness (nm) | Erosion amount (nm) | Transmittance change (%, wavelength range from 220 to 225 nm) | Film thickness (nm) | Erosion amount (nm) | Transmittance change (%, wavelength range from 220 to 225 nm) |
| 0 | 87.1 | - | - | 5 | - | - |
| 30 | 85.6 | 1.5 | -1.2 | 5 | 0 | 0.4 |
| 60 | 83.6 | 3.5 | -2.7 | 5 | 0 | 0.2 |
| 120 | 81.1 | 6 | -4.9 | 5 | 0 | 0.0 |
| 240 | 75.6 | 11.5 | -9.3 | 4.5 | 0.5 | -1.7 |

From Table 4, it is understood that the erosion amount can be reduced and the change in transmittance in a wavelength range from 220 nm to 225 nm can be suppressed in Example 8 with the outermost layer formed of the HfO₂ film as compared to Example 5 with the outermost layer formed of the SiO₂ film.

### Example 18

First, a synthetic quartz glass substrate (manufactured by USTRON Corporation) was prepared as a transparent substrate. Subsequently, a dielectric multilayer film was deposited by sputtering on a principal surface on one side of the prepared transparent substrate. Specifically, first, a hafnium target was sputtered using an argon gas and an oxygen gas as carrier gases, and thus a hafnium oxide film (HfO₂ film) was deposited on the principal surface on the one side of the transparent substrate. At this time, each of the flow rates of the argon gas and the oxygen gas was 100 sccm, and the target application power (power for deposition) was 4 kW. Subsequently, a silicon target was sputtered using the argon gas and the oxygen gas as the carrier gases, and thus a silicon oxide film (SiO₂ film) was deposited on the HfO₂ film. At this time, the flow rate of the argon gas and the oxygen gas was 100 sccm, and the target application power (power for deposition) was 4 kW. By repeating this operation, on the principal surface on the one side of the transparent substrate, the dielectric multilayer film including a film having a total of 38 layers was formed in which the HfO₂ film and the SiO₂ film were alternately laminated one layer by one layer, and thus the transparent substrate with the film was produced. The substrate temperature was maintained at room temperature (20°C) during the deposition. Subsequently, an optical filter was produced by heating the transparent substrate with the film at a temperature of 500°C for 60 minutes under atmospheric environment.

### Example 19

An optical filter was fabricated in the same manner as that in Example 18 except that, at the time of fabricating a transparent substrate with a film, the thickness of each layer and the number of laminated layers of the film were changed as depicted in Table 5 given below.

### Example 20

An optical filter was fabricated in the same manner as that in Example 18 except that, at the time of fabricating a transparent substrate with a film, the thickness of each layer and the number of laminated layers of the film were changed as depicted in Table 5 below, and the produced transparent substrate with the film was heated at a temperature of 550°C for 60 minutes under atmospheric environment.

### Example 21

An optical filter was fabricated in the same manner as that in Example 18 except that, at the time of fabricating a transparent substrate with a film, the thickness of each layer and the number of laminated layers of the film were changed as depicted in Table 5 below, and the produced transparent substrate with the film was heated at a temperature of 600°C for 60 minutes under atmospheric environment.

The following examples 22 to 25 do not fall under the scope of protection of the present invention.

### Example 22

An optical filter was fabricated in the same manner as that in Example 18 except that, at the time of fabricating a transparent substrate with a film, the thickness of each layer and the number of laminated layers of the film were changed as depicted in Table 5 below, and the produced transparent substrate with the film was heated at a temperature of 450°C for 60 minutes under atmospheric environment.

### Example 23

An optical filter was fabricated in the same manner as that in Example 22 except that, at the time of fabricating a transparent substrate with a film, the thickness of each layer and the number of laminated layers of the film were changed as depicted in Table 5 below, and the target application power (power for deposition) was 3.5 kW at the time of depositing the hafnium oxide film (HfO₂ film).

### Example 24

An optical filter was fabricated in the same manner as that in Example 22 except that, at the time of fabricating a transparent substrate with a film, the thickness of each layer and the number of laminated layers of the film were changed as depicted in Table 5 below, and the target application power (power for deposition) was 3 kW at the time of depositing the hafnium oxide film (HfO₂ film).

### Example 25

An optical filter was produced in the same manner as that in Example 23 except that, at the time of fabricating a transparent substrate with a film, the thickness of each layer and the number of laminated layers of the film were changed as depicted in Table 5 below.

**[Table 5]**

| Number of Layers from Transparent Substrate Side | Film Material | Example 18 | Example 19 | Example 20 | Example 21 | Example 22 | Example 23 | Example 24 | Example 25 |
|---|---|---|---|---|---|---|---|---|---|
| 1st Layer | HfO₂ | 35.6 nm | 6.5 nm | 25.3 nm | 33.9 nm | 17.2 nm | 6.4 nm | 16.7 nm | 35.6 nm |
| 2nd Layer | SiO₂ | 47.3 nm | 83.3 nm | 135.4 nm | 56.2 nm | 73.3 nm | 79.2 nm | 68.7 nm | 47.3 nm |
| 3rd Layer | HfO₂ | 30.1 nm | 12.7 nm | 21.8 nm | 25.9 nm | 10.3 nm | 8.5 nm | 20.9 nm | 30.1 nm |
| 4th Layer | SiO₂ | 47.8 nm | 129.4 nm | 49.7 nm | 59.4 nm | 64.0 nm | 142.7 nm | 54.9 nm | 47.8 nm |
| 5th Layer | HfO₂ | 29.0 nm | 31.2 nm | 23.2 nm | 36.5 nm | 11.8 nm | 13.4 nm | 22.5 nm | 29.0 nm |
| 6th Layer | SiO₂ | 65.1 nm | 37.4 nm | 53.5 nm | 62.4 nm | 59.8 nm | 63.4 nm | 60.3 nm | 65.1 nm |
| 7th Layer | HfO₂ | 41.9 nm | 35.2 nm | 19.7 nm | 26.4 nm | 18.9 nm | 15.4 nm | 25.2 nm | 41.9 nm |
| 8th Layer | SiO₂ | 50.9 nm | 32.5 nm | 60.8 nm | 56.2 nm | 55.6 nm | 63.7 nm | 65.8 nm | 50.9 nm |
| 9th Layer | HfO₂ | 29.8 nm | 36.8 nm | 14.5 nm | 27.6 nm | 21.3 nm | 12.6 nm | 25.3 nm | 29.8 nm |
| 10th Layer | SiO₂ | 46.7 nm | 17.3 nm | 56.0 nm | 188.3 nm | 53.4 nm | 65.8 nm | 59.5 nm | 46.7 nm |
| 11th Layer | HfO₂ | 29.5 nm | 34.6 nm | 21.4 nm | 25.8 nm | 21.8 nm | 7.9 nm | 22.9 nm | 29.5 nm |
| 12th Layer | SiO₂ | 55.8 nm | 40.5 nm | 49.6 nm | 51.4 nm | 52.5 nm | 63.8 nm | 56.2 nm | 55.8 nm |
| 13th Layer | HfO₂ | 46.2 nm | 30.8 nm | 24.3 nm | 24.9 nm | 21.5 nm | 15.0 nm | 21.7 nm | 46.2 nm |
| 14th Layer | SiO₂ | 55.0 nm | 37.6 nm | 48.1 nm | 48.7 nm | 52.8 nm | 58.5 nm | 55.3 nm | 55.0 nm |
| 15th Layer | HfO₂ | 29.0 nm | 32.3 nm | 24.7 nm | 24.5 nm | 20.8 nm | 18.7 nm | 19.7 nm | 29.0 nm |
| 16th Layer | SiO₂ | 44.7 nm | 33.5 nm | 48.4 nm | 49.3 nm | 53.3 nm | 56.5 nm | 131.2 nm | 44.7 nm |
| 17th Layer | HfO₂ | 28.7 nm | 34.9 nm | 24.2 nm | 23.1 nm | 20.8 nm | 18.8 nm | 13.5 nm | 28.7 nm |
| 18th Layer | SiO₂ | 42.8 nm | 27.2 nm | 49.5 nm | 52.0 nm | 53.1 nm | 58.4 nm | 61.3 nm | 42.8 nm |
| 19th Layer | HfO₂ | 29.0 nm | 35.9 nm | 23.3 nm | 20.1 nm | 21.3 nm | 15.4 nm | 16.7 nm | 29.0 nm |
| 20th Layer | SiO₂ | 40.3 nm | 28.8 nm | 50.3 nm | 54.0 nm | 53.4 nm | 62.2 nm | 60.4 nm | 40.3 nm |
| 21st Layer | HfO₂ | 29.0 nm | 34.2 nm | 22.9 nm | 20.6 nm | 21.0 nm | 10.9 nm | 16.7 nm | 29.0 nm |
| 22nd Layer | SiO₂ | 40.4 nm | 35.8 nm | 49.3 nm | 52.0 nm | 62.0 nm | 60.9 nm | 60.7 nm | 40.4 nm |
| 23rd Layer | HfO₂ | 29.1 nm | 31.9 nm | 23.9 nm | 22.7 nm | 15.9 nm | 17.6 nm | 14.2 nm | 29.1 nm |
| 24th Layer | SiO₂ | 42.3 nm | 39.2 nm | 48.8 nm | 50.5 nm | 194.6 nm | 56.1 nm | 133.7 nm | 42.3 nm |
| 25th Layer | HfO₂ | 28.8 nm | 31.3 nm | 24.4 nm | 230 nm | 22.8 nm | 20.9 nm | 16.2 nm | 28.8 nm |
| 26th Layer | SiO₂ | 41.9 nm | 46.4 nm | 50.1 nm | 51.2 nm | 62.3 nm | 54.8 nm | 61.2 nm | 41.9 nm |
| 27th Layer | HfO₂ | 28.2 nm | 37.4 nm | 24.2 nm | 21.6 nm | 22.2 nm | 21.7 nm | 16.1 nm | 28.2 nm |
| 28th Layer | SiO₂ | 41.9 nm | 65.5 nm | 63.8 nm | 55.2 nm | 66.6 nm | 60.7 nm | 65.7 nm | 41.9 nm |
| 29th Layer | HfO₂ | 28.4 nm | 33.7 nm | 7.7 nm | 17.1 nm | 32.2 nm | 26.7 nm | 8.1 nm | 28.4 nm |
| 30th Layer | SiO₂ | 42.2 nm | 44.7 nm | 122.2 nm | 59.8 nm | 67.3 nm | 72.7 nm | 140.9 nm | 42.2 nm |
| 31st Layer | HfO₂ | 29.2 nm | 31.0 nm | 26.3 nm | 14.7 nm | 24.9 nm | 24.4 nm | 8.1 nm | 29.2 nm |
| 32nd Layer | SiO₂ | 42.3 nm | 51.2 nm | 50.4 nm | 59.4 nm | 271.8 nm | 60.3 nm | 69.1 nm | 42.3 nm |
| 33rd Layer | HfO₂ | 28.6 nm | 30.7 nm | 260 nm | 18.2 nm | 20.4 nm | 21.1 nm | 15.6 nm | 28.6 nm |
| 34th Layer | SiO₂ | 42.0 nm | 67.2 nm | 49.8 nm | 57.8 nm | 69.7 nm | 62.3 nm | 248.8 nm | 42.0 nm |
| 35th Layer | HfO₂ | 32.4 nm | 34.8 nm | 27.2 nm | 18.3 nm | 14.9 nm | 23.2 nm | | 32.4 nm |
| 36th Layer | SiO₂ | 32.3 nm | 54.0 nm | 56.2 nm | 63.3 nm | 25.6 nm | 185.4 nm | | 32.3 nm |
| 37th Layer | HfO₂ | 35.8 nm | 29.8 nm | 33.0 nm | 9.8 nm | | 11.8 nm | | 35.8 nm |
| 38th Layer | SiO₂ | 83.2 nm | 20.4 nm | 69.7 nm | 71.5 nm | | 78.2 nm | | 83.2 nm |
| 39th Layer | HfO₂ | | | 31.1 nm | 6.2 nm | | 14.8 nm | | |
| 40th Layer | SiO₂ | | | 53.7 nm | 101.2 nm | | 24.9 nm | | |
| 41st Layer | HfO₂ | | | 33.9 nm | | | 33.9 nm | | |
| 42nd Layer | SiO₂ | | | 21.0 nm | | | 21.0 nm | | |
| Total of Layers (layers) | | 38 Layers | 38 Layers | 42 Layers | 40 Layers | 36 Layers | 42 Layers | 34 Layers | 38 Layers |

### Comparative Example 5

A dielectric multilayer film was deposited by sputtering on a principal surface on one side of a prepared transparent substrate in the same manner as that in Example 18. Specifically, first, an aluminum target was sputtered using an argon gas and an oxygen gas as carrier gases, and thus an aluminum oxide film (Al₂O₃ film) was deposited on the principal surface on the one side of the transparent substrate. At this time, the flow rate of the argon gas was 100 ccm, the flow rate of the oxygen gas was 20 ccm, and the target application power (power for deposition) was 4 kW. Subsequently, a silicon target was sputtered using the argon gas and oxygen gas as the carrier gases, and thus a silicon oxide film (SiO₂ film) was deposited on the Al₂O₃ film. At this time, each of the flow rates of the argon gas and the oxygen gas was 100 sccm, and the target application power (power for deposition) was 4 kW. By repeating this operation, on the principal surface on the one side of the transparent substrate, the dielectric multilayer film including a film having a total of 230 layers with a total thickness of 10 µm was formed in which the Al₂O₃ film and the SiO₂ film were alternately laminated one layer by one layer, and thus the transparent substrate with the film (optical filter) was produced. The substrate temperature was maintained at room temperature (20°C) during the deposition.

### Comparative Example 6

A dielectric multilayer film was deposited by sputtering on a principal surface on one side of a prepared transparent substrate in the same manner as that in Example 18. Specifically, first, a hafnium target was sputtered using an argon gas and an oxygen gas as carrier gases, and thus a hafnium oxide film (HfO₂ film) was deposited on the principal surface on the one side of the transparent substrate. At this time, each of the flow rates of the argon gas and the oxygen gas was 100 ccm, and the target application power (power for deposition) was 3 kW. Subsequently, a silicon target was sputtered using the argon gas and the oxygen gas as the carrier gases, and thus a silicon oxide film (SiO₂ film) was deposited on the HfO₂ film. At this time, the flow rate of the argon gas and the oxygen gas was 100 sccm, and the target application power (power for deposition) was 4 kW. By repeating this operation, on the principal surface on the one side of the transparent substrate, the dielectric multilayer film including a film having a total of 33 layers with a total thickness of 1700 nm was formed in which the HfO₂ film and the SiO₂ film were alternately laminated one layer by one layer, and thus the transparent substrate with the film was produced. The substrate temperature was maintained at room temperature (20°C) during the deposition. Subsequently, an optical filter was produced by heating the transparent substrate with the film at a temperature of 425°C for 60 minutes under atmospheric environment.

### Evaluation

### X-Ray Diffraction Measurement

The optical filters of Examples 18 to 25 and Comparative Examples 5 to 6 were subjected to X-ray diffraction measurement using a wide angle X-ray diffraction technique. As an X-ray diffractometer, "SmartLab", which is a product of Rigaku Corporation, was used. The measurement was performed using CuKα radiation (wavelength of 1.5418 Å) as a radiation source under the conditions of a scanning axis being 2θ/ω, a measurement range being 10 degrees to 65 degrees, a scanning rate being 2 degrees/min, a tube current being 200 mA, and a tube voltage being 45 kV.

Among the produced X-ray diffraction spectra, a spectrum in which the diffraction peak by the (1 1 1) crystal plane derived from a cubic hafnium oxide crystal was larger than the diffraction peak by the (-1 1 1) crystal plane derived from a monoclinic hafnium oxide crystal was evaluated as GOOD, and a spectrum in which the diffraction peak by the (1 1 1) crystal plane derived from a cubic hafnium oxide crystal was smaller than the diffraction peak by the (-1 1 1) crystal plane derived from a monoclinic hafnium oxide crystal was evaluated as POOR.

By using the produced X-ray diffraction spectra, the peak integrated intensity Ic of the diffraction peak by the (1 1 1) crystal plane derived from the cubic hafnium oxide crystal, the peak integrated intensity Im of the diffraction peak by the (-1 1 1) crystal plane derived from the monoclinic hafnium oxide crystal, and the ratio Ic/Im were determined.

### Spectral Transmittance

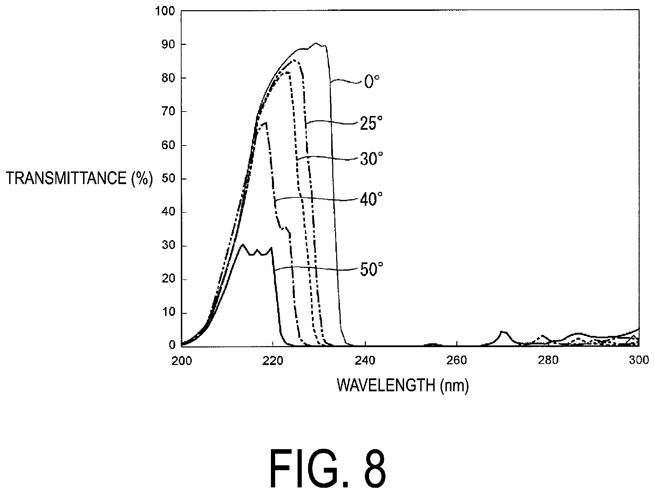

The spectral transmittance of each of the optical filters of Examples 18 to 25 and Comparative Examples 5 to 6 was measured using a spectral transmittance meter (manufactured by Hitachi High-Tech Corporation, Product Number "UH4150"). Specifically, the incident angle (AOI) was set to 0, 25, 30, 40, or 50 degrees, and the measurement wavelength was from 190 nm to 400 nm. An example of transmission spectra of an produced optical filter is depicted in FIG. 8.

FIG. 8 is a diagram illustrating transmission spectra at respective incident angles of the optical filter produced in Example 18. As illustrated in FIG. 8, in Example 18, it is understood that, even when the incident angle is increased, high spectral transmittance can be more effectively maintained at a wavelength of 222 nm and the maximum value of the spectral transmittance in a wavelength range from 237 nm to 280 nm can be further decreased.

Likewise, as for the optical filters of Examples 18 to 25 and Comparative Examples 5 to 6, the spectral transmittance at a wavelength of 222 nm and the spectral transmittance (maximum transmittance) in a wavelength range from 237 nm to 280 nm were measured with respective incident angles.

The measurement results are presented in Table 6 given below. In Table 6 below, a ratio of spectral transmittance T₃₀ at the wavelength of 222 nm with an incident angle of 30 degrees to spectral transmittance T₀ at the wavelength of 222 nm with an incident angle of 0 degrees (T₃₀/T₀) is additionally presented. In Table 6 below, the minimum transmittance in a wavelength range from 220 nm to 225 nm is also presented. A total thickness t_{H} of the high refractive index film (HfO₂ thickness), a total thickness t_{L} of the low refractive index film (SiO₂ thickness), and a film thickness ratio (t_{H}/t_{L}) are also presented.

**[Table 6]**

| | HfO₂ Thickness nm | SiO₂ Thickness nm | Film Thickness Ratio (t_{H}/t_{L}) | Spectral Transmittance at 222 nm (%) | | | | | Minimum Transmittance in Wavelength Range from 220 to 225 nm (%) | Maximum Transmittance Wavelength Range from 237 to 280 nm (%) | | | | Maximum Transmittance in Wavelength Range from 240 nm to 320 nm (%) | X-Ray Diffraction Measurement | |
|---|---|---|---|---|---|---|---|---|---|---|---|---|---|---|---|---|
| | | | | AOI = 0° | AOI = 30° | AOI = 40° | AOI = 50° | T₃₀/T₀ | AOI = 0° | AOI = 0° | AOI = 30° | AOI = 40° | AOI = 50° | AOI = 0° | Evaluation | Ic/Im |
| Example 18 | 598 | 905 | 0.661 | 82.6 | 80.1 | 34.7 | - | 0.97 | 77.8 | 0.5 | 0.2 | 3.3 | - | 3.2 | GOOD | 2.7 |
| Example 19 | 586 | 892 | 0.657 | 81.5 | 77.9 | 51.0 | 26.4 | 0.96 | 77.2 | 0.9 | 2.1 | 2.7 | 3.8 | 3.0 | GOOD | 2.7 |
| Example 20 | 503 | 1236 | 0.407 | 82.8 | 76.5 | 33.4 | - | 0.92 | 77.8 | 0.4 | 2.3 | 3.4 | - | 4.0 | GOOD | 2.6 |
| Example 21 | 441 | 1300 | 0.339 | 82.6 | 76.9 | 18.0 | - | 0.93 | 77.8 | 0.7 | 2.7 | 3.9 | - | 4.0 | GOOD | 2.5 |
| Example 22 | 360 | 1391 | 0.259 | 81.7 | 76.2 | 4.9 | 0.1 | 0.93 | 76.7 | 1.6 | 3.2 | 3.3 | 5.3 | 4.4 | GOOD | 1.3 |
| Example 23 | 359 | 1452 | 0.247 | 79.7 | 72.3 | 1.6 | - | 0.91 | 73.9 | 2.0 | 3.4 | 3.6 | - | 4.5 | GOOD | 1.1 |
| Example 24 | 300 | 1454 | 0.206 | 77.0 | 70.4 | 1.4 | - | 0.91 | 70.7 | 3.9 | 4.4 | 4.4 | - | 4.9 | POOR | 0.7 |
| Example 25 | 598 | 905 | 0.661 | 62.1 | 57.5 | 24.6 | 2.8 | 0.93 | 53.7 | 0.5 | 0.2 | 3.3 | 4.4 | 3.2 | GOOD | 1.1 |
| Comparative Example 5 | - | - | - | 75 | Several % | Several % | - | Below 0.5 | - | - | - | - | - | 17.8 | - | - |
| Comparative Example 6 | 240 | 1460 | 0.164 | 85 | 35 | Several % | - | 0.41 | - | - | - | - | - | 21.5 | POOR | 0.2 |

As is apparent from Table 6, it is understood that, even when the incident angle is increased, high spectral transmittance can be more effectively maintained at the wavelength of 222 nm and the maximum value of the spectral transmittance in a wavelength range from 237 nm to 280 nm can be further reduced in the optical filters of Examples 18 to 25 as compared to Comparative Examples 5 and 6.

In the optical filters of Examples 18 to 25, the maximum value of the spectral transmittance in a wavelength range from 240 nm to 320 nm was measured by the same method as that in Example 1, and the maximum value was found to be 5% or less. In Comparative Examples 5 and 6, the maximum values were 17.8% and 21.5%, respectively.

### Reference Signs List

1, 21 Optical filter
1a Principal surface
2 Transparent substrate
2a First principal surface
2b Second principal surface
3, 23 Dielectric multilayer film
4 High refractive index film
5 Low refractive index film
26 Outermost layer
31 Sterilization device
32 Housing
33 Light source
34 Sterilization target object

## Claims

1. An optical filter (1, 21), comprising:
a transparent substrate (2); and
a dielectric multilayer film (3, 23) provided on the transparent substrate (2) and containing hafnium oxide,
wherein the minimum value of spectral transmittance in a wavelength range from 220 nm to 225 nm is 50% or more with an incident angle of 0 degrees,
**characterized in that**
the maximum value of spectral transmittance in a wavelength range from 240 nm to 320 nm is 5% or less with an incident angle of 0 degrees, and
the dielectric multilayer film (3, 23) contains a cubic hafnium oxide crystal.

2. The optical filter (1, 21) according to claim 1,
wherein in X-ray diffraction measurement, a diffraction peak by a (1 1 1) crystal plane derived from a cubic hafnium oxide crystal is larger than a diffraction peak by a (-1 1 1) crystal plane derived from a monoclinic hafnium oxide crystal.

3. The optical filter (1, 21) according to claim 1 or 2,
wherein the dielectric multilayer film (3, 23) includes a high refractive index film (4) having a relatively high refractive index and a low refractive index film (5) having a relatively low refractive index, and
the high refractive index film (4) contains the hafnium oxide.

4. The optical filter (1, 21) according to claim 3,
wherein the low refractive index film (5) contains silicon oxide.

5. The optical filter (21) according to any one of claims 1 to 4,
wherein an outermost layer (26) of the dielectric multilayer film (23) is a film containing the hafnium oxide.

6. The optical filter (21) according to claim 5,
wherein a thickness of the outermost layer (26) is 1 nm or more and 10 nm or less.

7. The optical filter (1, 21) according to any one of claims 1 to 6,
wherein a ratio (T₃₀/T₀) of spectral transmittance T₃₀ at a wavelength of 222 nm with an incident angle of 30 degrees to spectral transmittance T₀ at a wavelength of 222 nm with an incident angle of 0 degrees is 0.5 or more.

8. The optical filter (1, 21) according to any one of claims 1 to 7,
wherein the dielectric multilayer film (3, 23) includes a high refractive index film (4) having a relatively high refractive index and a low refractive index film (5) having a relatively low refractive index, and
a ratio (t_{H}/t_{L}) of a total thickness t_{H} of the high refractive index film (4) to a total thickness t_{L} of the low refractive index film (5) is 0.2 or more.

9. The optical filter (1, 21) according to claim 8,
wherein the ratio (t_{H}/t_{L}) of the total thickness t_{H} of the high refractive index film (4) to the total thickness t_{L} of the low refractive index film (5) is 0.5 or more.

10. The optical filter (1, 21) according to any one of claims 1 to 9,
wherein with an incident angle of 0 degrees,
the minimum value of the spectral transmittance in a wavelength range from 220 nm to 225 nm is 50% or more, and
the maximum value of the spectral transmittance in a wavelength range from 237 nm to 280 nm is 10% or less.

11. The optical filter (1, 21) according to any one of claims 1 to 10,
wherein the maximum value of the spectral transmittance in a wavelength range from 237 nm to 280 nm is 20% or less with an incident angle of 40 degrees.

12. A method for producing an optical filter (1, 21) comprising: a transparent substrate (2); and a dielectric multilayer film (3, 23) provided on the transparent substrate (2) and containing hafnium oxide, wherein the minimum value of spectral transmittance in a wavelength range from 220 nm to 225 nm is 50% or more with an incident angle of 0 degrees, and the maximum value of spectral transmittance in a wavelength range from 240 nm to 320 nm is 5% or less with an incident angle of 0 degrees, the method comprising:
forming a transparent substrate (2) with a film by depositing a dielectric multilayer film (3, 23) on the transparent substrate (2) by sputtering method, the dielectric multilayer film (3, 23) containing hafnium oxide; and
heating the transparent substrate (2) with the film at a temperature of 500°C or higher.

13. The method for producing the optical filter (1, 21) according to claim 12,
wherein the temperature for the heating of the transparent substrate (2) with the film is 800°C or lower.

14. A sterilization device (31) for performing inactivation treatment on microorganisms to be treated, the sterilization device (31) comprising:
a light source (33) configured to emit light whose wavelength is in a wavelength range from 190 nm to 230 nm; and
the optical filter (1, 21) according to any one of claims 1 to 11.

## Patentansprüche

1. Optischer Filter (1, 21), umfassend:
ein transparentes Substrat (2) und
einen dielektrischen Mehrschichtfilm (3, 23), der auf dem transparenten Substrat (2) vorgesehen ist und Hafniumoxid enthält,
wobei der minimale Wert der spektralen Durchlässigkeit in einem Wellenlängenbereich von 220 nm bis 225 nm mit einem Einfallswinkel von 0 Grad 50 % oder mehr beträgt,
**dadurch gekennzeichnet, dass**
der maximale Wert der spektralen Durchlässigkeit in einem Wellenlängenbereich von 240 nm bis 320 nm mit einem Einfallswinkel von 0 Grad 5 % oder weniger beträgt und
der dielektrische Mehrschichtfilm (3, 23) einen kubischen Hafniumoxidkristall enthält.

2. Optischer Filter (1, 21) nach Anspruch 1,
wobei bei einer Röntgenbeugungsmessung ein Beugungspeak einer (1 1 1)-Kristallebene eines kubischen Hafniumoxidkristalls größer ist als ein Beugungspeak einer (-1 1 1)-Kristallebene eines monoklinen Hafniumoxidkristalls.

3. Optischer Filter (1, 21) nach Anspruch 1 oder 2,
wobei der dielektrische Mehrschichtfilm (3, 23) einen Film (4) mit hohem Brechungsindex und einen Film (5) mit niedrigem Brechungsindex umfasst, und
der Film (4) mit hohem Brechungsindex Hafniumoxid enthält.

4. Optischer Filter (1, 21) nach Anspruch 3,
wobei der Film (5) mit niedrigem Brechungsindex enthält.

5. Optischer Filter (21) nach einem der Ansprüche 1 bis 4,
wobei eine äußerste Schicht (26) des dielektrischen Mehrschichtfilms (23) ein Film ist, der Hafniumoxid enthält.

6. Optischer Filter (21) nach Anspruch 5,
wobei die Dicke der äußersten Schicht (26) 1 nm oder mehr und 10 nm oder weniger beträgt.

7. Optischer Filter (1, 21) nach einem der Ansprüche 1 bis 6,
wobei das Verhältnis (T₃₀/T₀) der spektralen Durchlässigkeit T₃₀ bei einer Wellenlänge von 222 nm mit einem Einfallswinkel von 30 Grad zur spektralen Durchlässigkeit T₀ bei einer Wellenlänge von 222 nm mit einem Einfallswinkel von 0 Grad 0,5 oder mehr beträgt.

8. Optischer Filter (1, 21) nach einem der Ansprüche 1 bis 7,
wobei der dielektrische Mehrschichtfilm (3, 23) einen Film (4) mit hohem Brechungsindex, der einen relativ hohen Brechungsindex aufweist, und einen Film (5) mit niedrigem Brechungsindex, der einen relativ niedrigen Brechungsindex aufweist, umfasst und
ein Verhältnis (t_{H}/t_{L}) der Gesamtdicke t_{H} des Films (4) mit hohem Brechungsindex zur Gesamtdicke t_{L} des Films (5) mit niedrigem Brechungsindex 0,2 oder mehr beträgt.

9. Optischer Filter (1, 21) nach Anspruch 8,
wobei ein Verhältnis (t_{H}/t_{L}) der Gesamtdicke t_{H} des Films (4) mit hohem Brechungsindex zur Gesamtdicke t_{L} des Films (5) mit niedrigem Brechungsindex 0,5 oder mehr beträgt.

10. Optischer Filter (1, 21) nach einem der Ansprüche 1 bis 9,
wobei mit einem Einfallswinkel von 0 Grad
der minimale Wert der spektralen Durchlässigkeit in einem Wellenlängenbereich von 220 nm bis 225 nm 50 % oder mehr beträgt und
der maximale Wert der spektralen Durchlässigkeit in einem Wellenlängenbereich von 237 nm bis 280 nm 10 % oder weniger beträgt.

11. Optischer Filter (1, 21) nach einem der Ansprüche 1 bis 10,
wobei der maximale Wert der spektralen Durchlässigkeit in einem Wellenlängenbereich von 237 nm bis 280 nm mit einem Einfallswinkel von 40 Grad 20 % oder weniger beträgt.

12. Verfahren zur Herstellung eines optischen Filters (1, 21), umfassend: ein transparentes Substrat (2) und einen dielektrischen Mehrschichtfilm (3, 23), der auf dem transparenten Substrat (2) vorgesehen ist und Hafniumoxid enthält, wobei der minimale Wert der spektralen Durchlässigkeit in einem Wellenlängenbereich von 220 nm bis 225 nm mit einem Einfallswinkel von 0 Grad 50 % oder mehr beträgt und der maximale Wert der spektralen Durchlässigkeit in einem Wellenlängenbereich von 240 nm bis 320 nm mit einem Einfallswinkel von 0 Grad 5 % oder weniger beträgt, das Verfahren umfassend:
Bilden eines transparenten Substrats (2) mit einem Film durch Abscheiden eines dielektrischen Mehrschichtfilms (3, 23) auf dem transparenten Substrat (2) durch Sputtern, wobei der dielektrische Mehrschichtfilm (3, 23) Hafniumoxid enthält, und
Erhitzen des transparenten Substrats (2) mit dem Film auf eine Temperatur von 500 °C oder mehr.

13. Verfahren zur Herstellung des optischen Filters (1, 21) nach Anspruch 12,
wobei die Temperatur zum Erhitzen des transparenten Substrats (2) mit dem Film 800 °C oder niedriger ist.

14. Sterilisationsvorrichtung (31) zur Ausführung einer Inaktivierungsbehandlung an zu behandelnden Mikroorganismen, die Sterilisationsvorrichtung (31) umfassend:
eine Lichtquelle (33), die dazu beschaffen ist, Licht zu emittieren, dessen Wellenlänge in einem Bereich von 190 nm bis 230 nm liegt, und
den optischen Filter (1, 21) nach einem der Ansprüche 1 bis 11.

## Revendications

1. Filtre optique (1, 21) comprenant :
un substrat transparent (2) ; et
un film multicouche diélectrique (3, 23) prévu sur le substrat transparent (2) et contenant de l'oxyde d'hafnium, filtre optique dans lequel la valeur minimum du facteur de transmission spectrale se situant dans une plage de longueurs d'onde allant de 220 nm à 225 nm est de 50 % ou plus avec un angle incident de 0 degré,
**caractérisé en ce que** la valeur maximum du facteur de transmission spectrale se situant dans une plage de longueurs d'onde allant de 240 nm à 320 nm est de 5 % ou moins avec un angle incident de 0 degré, et le film multicouche diélectrique (3, 23) contient un cristal cubique d'oxyde d'hafnium.

2. Filtre optique (1, 21) selon la revendication 1,
dans lequel, lors d'une mesure de diffraction des rayons X, un pic de diffraction formé par un plan de cristal (1 1 1) obtenu à partir d'un cristal cubique d'oxyde d'hafnium est plus grand qu'un pic de diffraction formé par un plan de cristal (-1 1 1) obtenu à partir d'un cristal monoclinique d'oxyde d'hafnium.

3. Filtre optique (1, 21) selon la revendication 1 ou 2,
dans lequel le film multicouche diélectrique (3, 23) comprend un film (4) à indice de réfraction élevé, ledit film multicouche ayant un film (5) à indice de réfraction relativement élevé et à faible indice de réfraction, ledit dernier film ayant un indice de réfraction relativement faible, et
le film (4) à indice de réfraction élevé contient de l'oxyde d'hafnium.

4. Filtre optique (1, 21) selon la revendication 3,
dans lequel le film (5) à faible indice de réfraction contient de l'oxyde de silicium.

5. Filtre optique (21) selon l'une quelconque des revendications 1 à 4,
dans lequel une couche (26) située le plus à l'extérieur du film multicouche diélectrique (23) est un film contenant de l'oxyde d'hafnium.

6. Filtre optique (21) selon la revendication 5,
dans lequel une épaisseur de la couche (26) située le plus à l'extérieur est de 1 nm ou plus et de 10 nm ou moins.

7. Filtre optique (1, 21) selon l'une quelconque des revendications 1 à 6,
dans lequel un rapport (T₃₀/T₀) du facteur de transmission spectrale T₃₀ à une longueur d'onde de 222 nm avec un angle incident de 30 degrés, relativement au facteur de transmission spectrale T₀ à une longueur d'onde de 222 nm avec un angle incident de 0 degré, est de 0,5 ou plus.

8. Filtre optique (1, 21) selon l'une quelconque des revendications 1 à 7,
dans lequel le film multicouche diélectrique (3, 23) comprend un film (4) à indice de réfraction élevé, ledit film multicouche ayant un film (5) à indice de réfraction relativement élevé et à faible indice de réfraction, ledit dernier film ayant un indice de réfraction relativement faible, et
un rapport (t_{H}/t_{L}) d'une épaisseur totale t_{H} du film (4) à indice de réfraction élevé, relativement à une épaisseur totale t_{L} du film (5) à faible indice de réfraction, est de 0,2 ou plus.

9. Filtre optique (1, 21) selon la revendication 8,
dans lequel le rapport (t_{H}/t_{L}) de l'épaisseur totale t_{H} du film (4) à indice de réfraction élevé, relativement à l'épaisseur totale t_{L} du film (5) à faible indice de réfraction, est de 0,5 ou plus.

10. Filtre optique (1, 21) selon l'une quelconque des revendications 1 à 9,
dans lequel, avec un angle incident de 0 degré,
la valeur minimum du facteur de transmission spectrale se situant dans une plage de longueurs d'onde allant de 220 nm à 225 nm est de 50 % ou plus, et
la valeur maximum du facteur de transmission spectrale se situant dans une plage de longueurs d'onde allant de 237 nm à 280 nm est de 10 % ou moins.

11. Filtre optique (1, 21) selon l'une quelconque des revendications 1 à 10,
dans lequel la valeur maximum du facteur de transmission spectrale se situant dans une plage de longueurs d'onde allant de 237 nm à 280 nm est de 20 % ou moins avec un angle incident de 40 degrés.

12. procédé de production d'un filtre optique (1, 21) comprenant : un substrat transparent (2) ; et un film multicouche diélectrique (3, 23) prévu sur le substrat transparent (2) et contenant de l'oxyde d'hafnium, procédé dans lequel la valeur minimum du facteur de transmission spectrale se situant dans une plage de longueurs d'onde allant de 220 nm à 225 nm est de 50 % ou plus avec un angle incident de 0 degré, et la valeur maximum du facteur de transmission spectrale se situant dans une plage de longueurs d'onde allant de 240 nm à 320 nm est de 5 % ou moins avec un angle incident de 0 degré, le procédé consistant :
à former un substrat transparent (2) avec un film en déposant un film multicouche diélectrique (3, 23) sur le substrat transparent (2) par un procédé de pulvérisation, le film multicouche diélectrique (3, 23) contenant de l'oxyde d'hafnium ; et
à chauffer le substrat transparent (2) et le film à une température égale ou supérieure à 500°C.

13. procédé de production du filtre optique (1, 21) selon la revendication 12,
procédé dans lequel la température pour le chauffage du substrat transparent (2) et du film est égale ou inférieure à 800°C.

14. Dispositif de stérilisation (31) pour effectuer un traitement d'inactivation sur des microorganismes devant être traités, le dispositif de stérilisation (31) comprenant :
une source de lumière (33) configurée pour émettre de la lumière dont la longueur d'onde est dans une plage de longueurs d'onde allant de 190 nm à 230 nm ; et
le filtre optique (1, 21) selon l'une quelconque des revendications 1 à 11.
